# EUROPEAN PATENT SPECIFICATION

(11) **EP 1 098 617 B1**
(45) Date of publication and mention of the grant of the patent: **09.04.2003**
(21) Application number: 99934098.7
(22) Date of filing: 16.07.1999
(51) Int. Cl.: A61F 13/15

(54) **APPARATUS FOR TRANSPORTING A CONTINUOUS WEB, AND FOR MANIPULATING THE WEB**
VORRICHTUNG ZUM TRANSPORTIEREN UND MANIPULIEREN VON EINER MATERIALBAHN
APPAREIL POUR LE TRANSPORT D'UNE BANDE CONTINUE ET POUR LA MANIPULATION DE LA BANDE

(30) Priority: 22.07.1998 EP 98113667
(43) Date of publication of application: 16.05.2001
(73) Proprietor: THE PROCTER & GAMBLE COMPANY, Cincinnati, Ohio 45202 (US)
(72) Inventor: KUNTZE, Lothar, D-53359 Rheinbach (DE); SCHMITZ, Christoph, Johann, D-53881 Euskirchen-Stotzheim (DE)
(74) Representative: Hirsch, Uwe Thomas
(86) International application number: US9916138
(87) International publication number: WO00004855

(56) References cited:
- EP-A- 0 451 705
- US-A- 4 081 301
- US-A- 4 227 952
- US-A- 4 488 923
- US-A- 4 610 751

## Description

The present invention relates to an apparatus for transporting a continuous web, and for manipulating the web especially to form web loops. The apparatus is particularly useful in the manufacture of disposable absorbent articles, including diapers, adult incontinence products, sanitary napkins and the like.

Manufacturing processes are often required to provide discrete strips of a material onto a continuous web, in such a way that the discrete webs of material are spaced apart along the length of the continuous web. Features manufactured in this way include elastic strips, one example of which is the elastic leg cuffs applied to diapers.

US-A-4 081 301, issued on March 1978, discloses a process for applying a material, such as an elastic material, to a continuous web. The material is only glued along the length of the web in discrete sections, so that when the web is cut the elastic material remains extended only in the discrete section in which it has been glued, and contracts elsewhere.

US-A-4 227 952, issued on 14^{th} October 1980, discloses a conveyor which carries spaced web support plates and wherein a continuous web is tucked in between the support plates to form web loops is known. Various features, such as leg elastic, are then applied to the web which lies on the web support plate, but not to the web loop. This forming process requires less elastic material and provides material savings.

EP 451 705 discloses an apparatus for securing elastic to a flexible backing for use in catamenial pads.

US 4, 610, 751 discloses an apparatus for the separation of strip sections from a moving material web. The apparatus comprises a multiplicity of conveying elements rotating on the circular periphery, which have a distance to one another which in the peripheral direction changes during each rotation. The apparatus can be used to separate strip sections from each other.

An apparatus of this type comprises a plurality of web support plates, each web support plate having an outer web support surface. However the apparatus is mechanically complex and not well-suited to high speed manufacturing machines.

The object of the present invention is to provide a mechanically simpler and more reliable apparatus for applying features, including elastic features, to a continuous web, in particular on a high speed manufacturing machine.

A further object of the invention is to provide a process for transporting and manipulating a web using the apparatus of the invention.

### Summary of the Invention

The object of the invention is achieved by an apparatus for transporting a continuous web, and for manipulating the web, the apparatus comprising a plurality of web support plates, each web support plate having an outer web support surface, the apparatus further comprising a means for forming web loops between adjacent web support plates, wherein each of the web support surfaces lies in an arc, each of the arcs lying around the circumference of a circular path, and wherein the web support surfaces and means for forming web loops are rotatably mounted about the principal axis (25) of the circular path, and wherein the means for forming web loops is provided by rotatably driving the web support plates around the circular path with a circumferential velocity (V), wherein the circumferential velocity (V) is varied between a minimum circumferential velocity (Vₘᵢₙ) and a maximum circumferential velocity (Vₘₐₓ) so that the distance (d) between adjacent web support plates varies between a minimum distance and a maximum distance.

The invention also relates to a process for transporting a continuous web around the apparatus whereby the distance between adjacent web support plates is greater than the minimum distance, and wherein the web is transported around the circular path so that a web loop is formed when the distance between adjacent web support plates decreases towards the minimum distance.

### Brief Description of the Drawings

Figure 1 shows a diagrammatic representation of a cross-section through the apparatus of the present invention.
Figure 2 shows a more detailed cross-section through the apparatus of the present invention, including linkages between adjacent web support plates.
Figure 3 shows a representation in perspective of the path of a continuous web as it passes around the apparatus of the present invention in a first embodiment of the process of the invention.
Figure 4 shows a representation in perspective of the path of a continuous web as it passes around the apparatus of the present invention in a second embodiment of the process of the invention.
Figure 5 shows a representation in perspective of the path of a continuous web as it passes around the apparatus of the present invention in a third embodiment of the process of the invention. Figure 5a shows the profile of a curved elastic feature applied to the continuous web.

### Detailed Description of the Invention

It will be readily apparent to those skilled in the art that although the following description of the present invention is in connection with a single use diaper structure having discrete elastic regions or strips, the present invention may be practiced with equal facility on nearly any web.

In the following description a "continuous web" is a web of material which is continuous in the machine direction. A preferred continuous web comprises a plurality of interconnected single use disposable absorbent articles, such as diapers. Typically, each diaper is comprised of an absorbent pad element or absorbent core, and elastomeric elements or patches. The absorbent pad elements and the elastomeric elements are located between a backsheet and a topsheet, or alternatively, on top of a backsheet or topsheet. The continuous webs of backsheet material and topsheet material are preferably maintained under very slight tension in the machine direction to prevent wrinkling and to facilitate registration with the diaper assembly and converting operations until the completed diaper web is severed into discrete diapers by cutting across the width of the web.

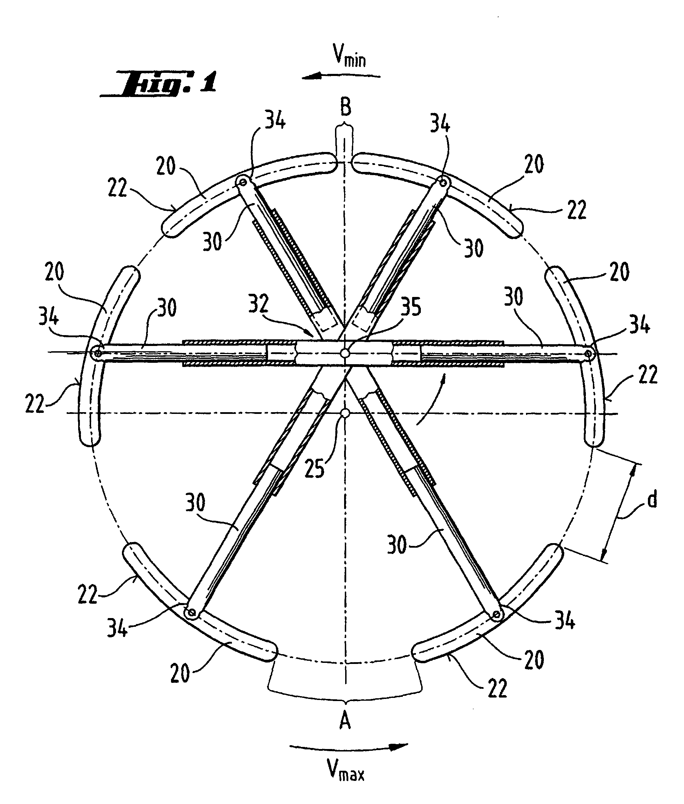

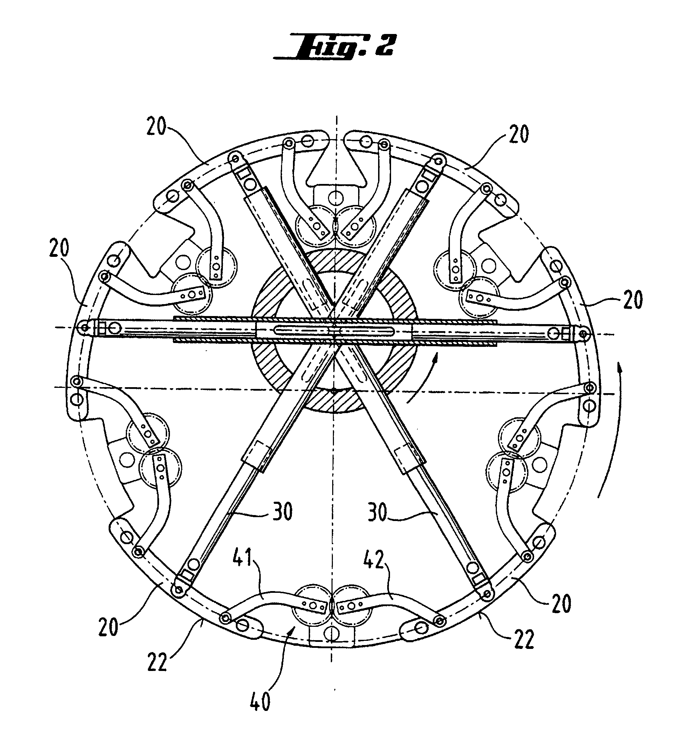

Figures 1 and 2 illustrate a cross-section through a preferred embodiment of the apparatus of the present invention. An apparatus having six web support plates 20 is illustrated, each web support plate comprises a web support surface 22 facing outwardly, and the web support plates 20 are rotated so that the web support surfaces 22 trace out an essentially circular path. The apparatus will now be described with reference to one of the web support plates as it moves around the circular path illustrated in Figure 1. An anti-clockwise rotation is illustrated, but the invention could be equally well put into practice with a clockwise rotation. As the web support plate 20 passes through point A of the circular path (at the bottom of the circular path as illustrated in Figure 1), it has a maximum circumferential velocity Vₘₐₓ. As the web support plate 20 is rotated towards the top of the circular path it is decelerated until it reaches a minimum circumferential velocity Vₘᵢₙ at point B of the circular path. As the web support plate 20 continues around the circular path it slows down until it returns to point A. Adjacent web support plates are spaced apart by a distance d. The adjacent web support plates either side of point A in Figure 1 have a maximum distance d between them. As the web support plates are rotated one of the web support plates has a faster circumferential velocity than the adjacent web support plate, and the faster web support plate catches up with the slower web support plate, thereby reducing the distance d between them. When the adjacent web support plates lie either side of point B in Figure 1 the distance between them is a minimum. It is preferred that the circumferential velocity V of the web support plates 20 varies according to a sinusoidal function, and wherein one cycle of the sinusoidal function corresponds to one complete rotation of the web support plate 20 around the circular path.

Consequently a continuous web 100 placed against the web support plates is transported around the apparatus and furthermore the continuous web 100 is manipulated in such a way that a web loop 101 is formed between adjacent web support plates 20 as the adjacent web support plates 20 move closer together.

In a particularly preferred embodiment of the invention the circumferential velocity of the web support plates is controlled by mechanical means. In Figure 1 the mechanical means comprises six extendible arms 30, that is to say one extendible arm 30 to drive each of the web support plates 20. Each extendible arm 30 has a proximal end 32 and a distal end 34, the proximal end 32 of each extendible arm 30 being mounted on a second axis of rotation 35 and the distal end 34 of each extendible arm 30 being pivotally mounted on each web support plate 20. The principal axis 25 and the second axis 35 are parallel but off-set in relation to each other, so that the extendible arms 30 drive the web support plates 20 around the circular path with the variable circumferential velocity.

Figure 2 shows a similar schematic representation to Figure 1, and additionally Figure 2 also illustrates preferred linking means 40. Adjacent web support plates 20 are pivotally connected to each other by linking means 40. The linking means illustrated comprise a first link 41 and a second link 42. The first link 41 and the second link 42 are pivotally connected to each other near one end of the links, the other end of each link being pivotally connected near to the ends of adjacent web support plates 20.

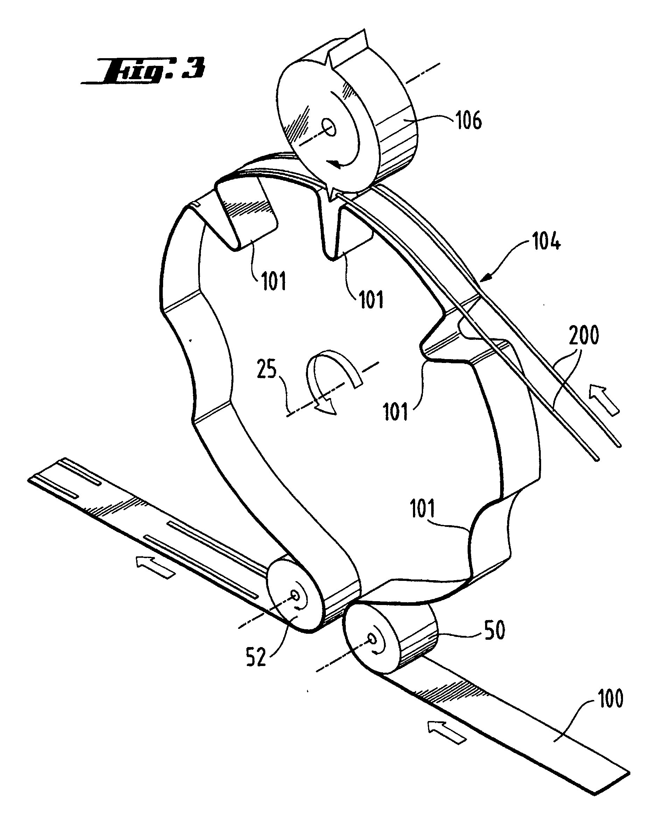

Figure 3 illustrates the path of a continuous web 100 as it passes around the apparatus of the present invention in a first embodiment of the process of the invention. The web is fed to a transfer roll 50 by a feeding means (not shown) and is transferred to the web support surface of the apparatus. The apparatus itself is not shown in Figure 3 for clarity. As the web is drawn around the circular path by means of the web support plates (not shown), web loops 101 are formed as described hereinabove. Figure 3 also shows a pair of elastic strips 200 being fed by a feeding means (not shown) to application points 104. At the application points 104 the elastic strips 200 are attached to the continuous web 100. The means of attachment is not important in this invention and may be achieved by glue, hot melt, self-adhesive material or any other means. Figure 3 also shows a cutting station 106 at which the elastic strips 200 are cut into discrete strips. The continuous web 100 is not necessarily cut at the cutting station 106, indeed it is preferred that the continuous web 100 is not cut on the apparatus of the present invention. The continuous web 100 is not cut because the cutting station 106 is adjacent to a web loop 101 as shown. Subsequently the web 100 accelerates around the apparatus, thereby releasing the web loops 101, and the continuous web with discrete lengths of elastic material adhered to it, the discrete lengths of elastic material being spaced apart, is peeled off the web support surface at a second transfer roll 52.

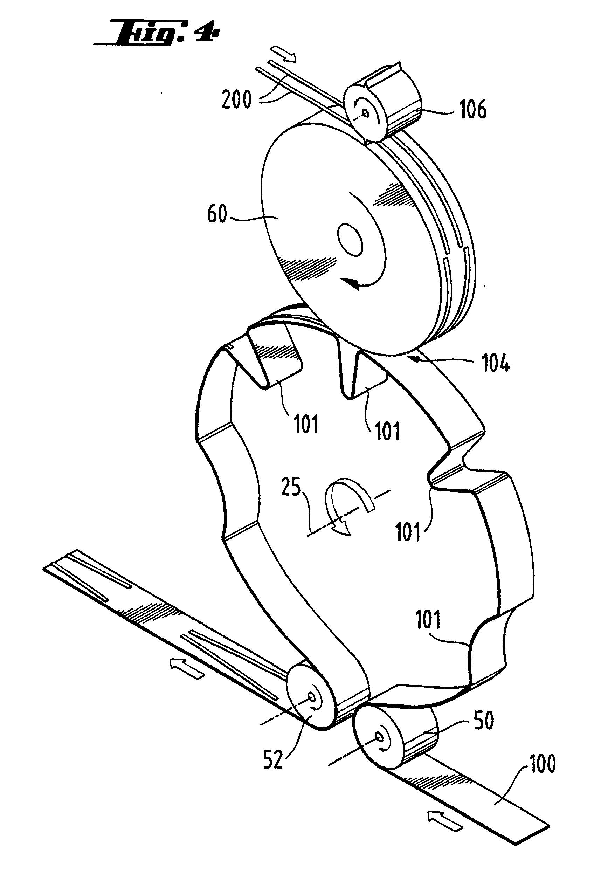

Figure 4 illustrates the path of a continuous web 100 as it passes around the apparatus of the present invention in a second embodiment of the process of the invention. The web is fed to a transfer roll 50 by a feeding means (not shown) and is transferred to the web support surface of the apparatus. The apparatus itself is not shown in Figure 4 for clarity. As the web is drawn around the circular path by means of the web support plates (not shown), web loops 101 are formed as described hereinabove.

In Figure 4 the elastic strips 200 are provided on the surface of an applicator roll 60. The elastic strips 200 are cut and deflected on the surface of the applicator roll 60 prior to the application point 104.

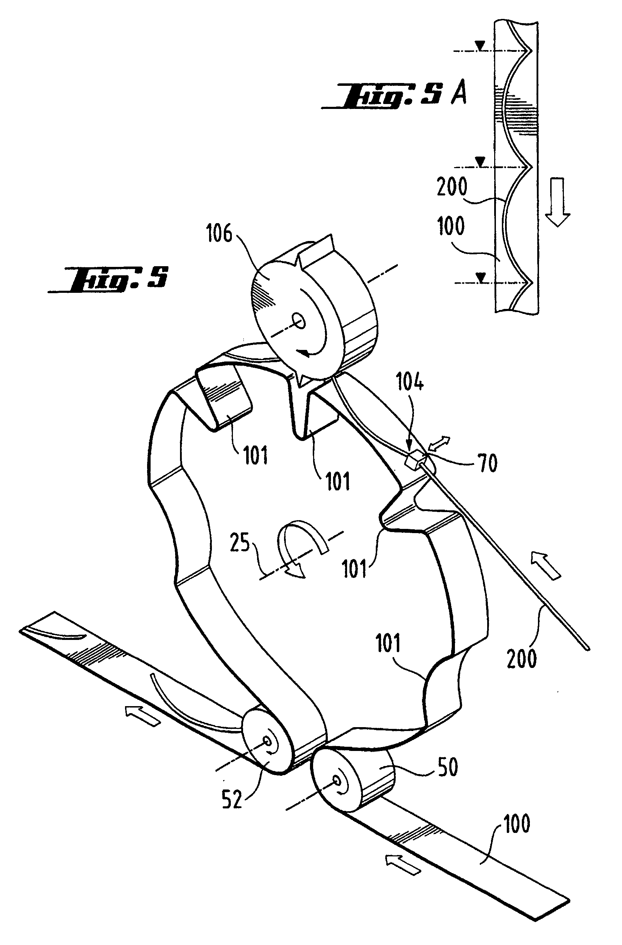

Figure 5 illustrates the path of a continuous web 100 as it passes around the apparatus of the present invention in a third embodiment of the process of the invention. The web is fed to a transfer roll 50 by a feeding means (not shown) and is transferred to the web support surface of the apparatus. The apparatus itself is not shown in Figure 5 for clarity. As the web is drawn around the circular path by means of the web support plates (not shown), web loops 101 are formed as described hereinabove.

In Figure 5 a deflection means 70 is provided in order to apply an elastic strip 200 in a curved profile. The deflection means 70 is adjacent to the application point 104 moves in the cross-machine direction with a frequency corresponding to the speed of the cycle of the web support plates 20.

Figure 5a shows the profile of a curved elastic feature applied to the continuous web.

## Claims

1. An apparatus (10) for transporting a continuous web (100), and for manipulating the web (100), the apparatus comprising a plurality of web support plates (20), each web support plate (20) having an outer web support surface (22), the apparatus further comprising a means for forming web loops (101) between adjacent web support plates (20), wherein each of the web support surfaces (22) lies in an arc, each of the arcs lying around the circumference of a circular path, and wherein the web support surfaces (22) and means for forming web loops (101) are rotatably mounted about the principal axis (25) of the circular path **characterized in that** the means for forming web loops (101) is provided by rotatably driving the web support plates (20) around the circular path with a circumferential velocity (V), wherein the circumferential velocity (V) is varied between a minimum circumferential velocity (Vₘᵢₙ) and a maximum circumferential velocity (Vₘₐₓ) so that the distance (d) between adjacent web support plates (20) varies between a minimum distance and a maximum distance.

2. An apparatus (10) according to claim 1 wherein the circumferential velocity (V) of the web support plates (20) varies according to a sinusoidal function, and wherein one cycle of the sinusoidal function corresponds to one complete rotation of the web support plate (20) around the circular path.

3. An apparatus (10) according to claim 2 wherein each web support plate (20) is driven around the circular path by an extendible arm (30), the proximal end (32) of each extendible arm (30) being mounted on a second axis of rotation (35) and the distal end (34) of each extendible arm (30) being pivotally mounted on each web support plate (20), and wherein the principal axis (25) and the second axis (35) are parallel but off-set in relation to each other, so that the extendible arms (30) drive the web support plates (20) around the circular path with the variable circumferential velocity (V).

4. An apparatus (10) according to any of claims 1 to 3 wherein adjacent web support plates (20) are pivotally connected to each other by linking means (40).

5. A process for transporting a continuous web (100), and for manipulating the web, wherein the web is placed against the web support plates (20) of the apparatus (10) of any of claims 1 to 4 at a position where the distance (d) between adjacent web support plates (20) is greater than the minimum distance, and wherein the web (100) is transported around the circular path so that a web loop (101) is formed when the distance (d) between adjacent web support plates (20) decreases towards the minimum distance.

6. A process according to claim 5 for applying a material (200) to the continuous web (100) wherein the material (200) is applied to the web (100) at an application point (104) which is adjacent to the circular path of the web support plates (20), so that the material (200) is applied to that part of the web lying upon one of the web support plates (20), but the material (200) is not applied to the remaining part of the web which is in the web loop (101).

7. A process according to claim 6 wherein the material is cut at a cutting station (106) so that the material (200) remains attached to the continuous web (100) only in discrete sections, each section corresponding to the part of the web lying adjacent to the web support plate (20).

8. A process according to either of claims 6 or 7 wherein the material (100) is an elastic material, preferably an elastic material applied under tension at the application point (104).

## Patentansprüche

1. Vorrichtung (10) zum Fördern einer kontinuierlichen Bahn (100) und zur Handhabung der Bahn (100), wobei die Vorrichtung eine Vielzahl von Bahn-Träger-Platten (20) umfasst, wobei jede Bahn-Träger-Platte (20) eine äußere Bahn-Träger-Oberfläche (22) aufweist, wobei die Vorrichtung ferner ein Mittel zum Bilden von Bahn-Schlaufen (101) zwischen benachbart angeordneten Bahn-Träger-Platten (20) umfasst, wobei jede der Bahn-Träger-Oberflächen (22) auf einem Bogen liegt, wobei jeder der Bögen den Umfang einer kreisförmigen Bahn liegt, und wobei die Bahn-Träger-Oberflächen (22) und das Mittel zum Bilden von Bahn-Schlaufen (101) drehbar um die Haupt-Achse (25) der kreisförmigen Bahn angebracht sind, **dadurch gekennzeichnet, dass** das Mittel zum Bilden von Bahn-Schlaufen (101) durch Dreh-Antreiben der Bahn-Träger-Platten (20) um die kreisförmige Bahn mit einer Umfangs-Geschwindigkeit (V) gebildet ist, wobei die Umfangs-Geschwindigkeit (V) zwischen einer Mindest-Umfangsgeschwindigkeit (Vₘᵢₙ) und einer Höchst-Umfangsgeschwindigkeit (Vₘₐₓ) variiert wird, sodass der Abstand (d) zwischen benachbart angeordneten Bahn-Träger-Platten (20) zwischen einem Mindest-Abstand und einem Höchst-Abstand variiert.

2. Vorrichtung (10) gemäß Anspruch 1, wobei die Umfangs-Geschwindigkeit (V) der Bahn-Träger-Platten (20) gemäß einer Sinus-Funktion variiert, und wobei eine Periode der Sinus-Funktion einer vollständigen Drehung der Bahn-Träger-Platte (20) um die kreisförmige Bahn entspricht.

3. Vorrichtung (10) gemäß Anspruch 2, wobei jede Bahn-Träger-Platte (20) um die kreisförmige Bahn durch einen ausziehbaren Arm (30) angetrieben wird, wobei das proximale Ende (32) von jedem ausziehbaren Arm (30) auf einer zweiten Drehachse (35) angebracht ist und das distale Ende (34) von jedem ausziehbaren Arm (30) gelenkig an jeder Bahn-Träger-Platte (20) angebracht ist, und wobei die Haupt-Achse (25) und die zweite Achse (35) parallel, aber versetzt zueinander sind, sodass die ausziehbaren Arme (30) die Bahn-Träger-Platten (20) um die kreisförmige Bahn mit der veränderlichen Geschwindigkeit (V) antreiben.

4. Vorrichtung (10) gemäß einem der Ansprüche 1 bis 3, wobei benachbart angeordnete Bahn-Träger-Platten (20) gelenkig miteinander durch Verbindungsmittel (40) verbunden sind.

5. Verfahren zum Fördern einer kontinuierlichen Bahn (100) und zum Bearbeiten der Bahn, wobei die Bahn an den Bahn-Träger-Platten (20) der Vorrichtung (10) gemäß den Ansprüchen 1 bis 4 an einer Stelle positioniert ist, an welcher der Abstand (d) zwischen benachbart angeordneten Bahn-Träger-Platten (20) größer als der Mindest-Abstand ist, und wobei die Bahn (100) derart um die kreisförmige Bahn gefördert wird, dass eine Bahn-Schlaufe (101) gebildet wird, wenn sich der Abstand (d) zwischen benachbart angeordneten Bahn-Träger-Platten (20) auf den Mindest-Abstand zu reduziert.

6. Verfahren gemäß Anspruch 5 zum Anbringen eines Materials (200) an der kontinuierlichen Bahn (100), wobei das Material (200) an der Bahn (100) an einem Anbringungs-Punkt (104) angebracht wird, welcher benachbart zu der kreisförmigen Bahn der Bahn-Träger-Platten (20) ist, sodass das Material (200) an dem auf einem der Bahn-Träger-Platten (20) liegenden Abschnitt der Bahn angebracht wird, aber das Material (200) nicht auf dem verbleibenden Abschnitt der Bahn angebracht wird, der sich in der Bahn-Schlaufe (101) befindet.

7. Verfahren gemäß Anspruch 6, wobei das Material an einer SchneideStation (106) derart geschnitten wird, dass das Material (200) an der kontinuierlichen Bahn (100) nur in einzelnen Teilabschnitten befestigt bleibt, wobei jeder Teilabschnitt dem Abschnitt der Bahn entspricht, der benachbart zu der Bahn-Träger-Platte (20) liegt.

8. Verfahren gemäß Anspruch 6 oder 7, wobei das Material (100) ein elastisches Material, vorzugsweise ein unter Spannung an dem Anbringungs-Punkt (104) angebrachtes, elastisches Material, ist.

## Revendications

1. Appareil (10) destiné à transporter une nappe continue (100), et à manipuler la nappe (100), l'appareil comprenant une pluralité de plaques de support de nappe (20), chaque plaque de support de nappe (20) présentant une surface de support de nappe (22) extérieure, l'appareil comprenant, en outre, un moyen pour former des boucles de nappe (101) entre les plaques de support de nappe (20) adjacentes, dans lequel chacune des surfaces de support de nappe (22) se trouve dans un arc, chacun des arcs se trouvant autour de la circonférence d'un trajet circulaire, et dans lequel les surface de support de nappe (22) et le moyen pour former des boucles de nappe (101 sont montés à rotation autour de l'axe principal (25) du trajet circulaire,
**caractérisé en ce que** le moyen pour former des boucles de nappe (101) est fourni en entraînant de manière rotative les plaques de support de nappe (20) autour du trajet circulaire à une certaine vitesse circonférentielle (V), la vitesse circonférentielle (V) variant entre une vitesse circonférentielle minimale (Vₘᵢₙ) et une vitesse circonférentielle maximale (Vₘₐₓ) afin que la distance (d) entre les plaques de support de nappe (20) adjacentes varie entre une distance minimale et une distance maximale.

2. Appareil (10) selon la revendication 1, dans lequel la vitesse circonférentielle (V) des plaques de support de nappe (20) varie selon une fonction sinusoïdale, et dans lequel un cycle de la fonction sinusoïdale correspond à une rotation complète de la plaque de support de nappe (20) autour du trajet circulaire.

3. Appareil (10) selon la revendication 2, dans lequel chaque plaque de support de nappe (20) est entraînée autour du trajet circulaire par un bras extensible (30), l'extrémité proximale (32) de chaque bras extensible (30) étant montée sur un deuxième axe de rotation (35), et l'extrémité distale (34) de chaque bras extensible (30) étant montée à pivotement sur chaque plaque de support de nappe (20), et dans lequel l'axe principal (25) et le deuxième axe (35) sont parallèles, mais décalés selon une relation l'un à l'autre, de sorte que les bras extensibles (30) entraînent les plaques de support de nappe (20) autour du trajet circulaire à la vitesse circonférentielle (V) variable.

4. Appareil (10) selon l'une quelconque des revendications 1 à 3, dans lequel les plaques de support de nappe (20) adjacentes sont reliées à pivotement les unes aux autres par des moyens de liaison (40).

5. Procédé pour transporter une nappe continue (100) et pour manipuler la nappe, dans lequel la nappe est placée contre les plaques de support de nappe (20) de l'appareil (10) selon l'une quelconque des revendications 1 à 4 en une position où la distance (d) entre les plaques de support de nappe (20) adjacentes est supérieure à la distance minimale, et dans lequel la nappe (100) est transportée autour du trajet circulaire afin qu'une boucle de nappe (101) soit formée lorsque la distance (d) entre les plaques de support de nappe (20) adjacentes décroît en direction de la distance minimale.

6. Procédé selon la revendication 5 pour appliquer un matériau (200) à la nappe continue (100), dans lequel le matériau (200) est appliqué à la nappe (100) en un point d'application (104) qui est adjacent au trajet circulaire des plaques de support de nappe (20), afin que le matériau (200) soit appliqué à la partie de la nappe se trouvant sur une des plaques de support de nappe (20), mais le matériau (200) n'est pas appliqué à la partie restante de la nappe qui est dans la boucle de nappe (101).

7. Procédé selon la revendication 6, dans lequel le matériau est découpé à un poste de découpe (106) afin que le matériau (200) reste fixé à la nappe continue (100) seulement en des parties distinctes, chaque partie correspondant à la partie de la nappe qui est adjacente à la plaque de support de nappe (20).

8. Procédé selon la revendication 6 ou la revendication 7, dans lequel le matériau (100) est un matériau élastique, de préférence, un matériau élastique appliqué sous tension au niveau du point d'application (104).
